(19) **Europäisches Patentamt**
**European Patent Office**
**Office européen des brevets**

(11) **EP 3 254 094 B1**

(12) **EUROPÄISCHE PATENTSCHRIFT**

(45) Veröffentlichungstag und Bekanntmachung des Hinweises auf die Patenterteilung:
**28.06.2023 Patentblatt 2023/26**

(21) Anmeldenummer: **16702520.4**

(22) Anmeldetag: **29.01.2016**

(51) Internationale Patentklassifikation (IPC):
**G01N 15/10** (2006.01)   **G01N 22/00** (2006.01)
**G01N 33/487** (2006.01)

(52) Gemeinsame Patentklassifikation (CPC):
**G01N 22/00; G01N 15/1031;** G01N 33/48728

(86) Internationale Anmeldenummer:
**PCT/EP2016/051887**

(87) Internationale Veröffentlichungsnummer:
**WO 2016/124489 (11.08.2016 Gazette 2016/32)**

(54) **INHOMOGENE ÜBERTRAGUNGSLEITUNG ZUR POSITIONSAUFGELÖSTEN PERMITTIVITÄTSBESTIMMUNG EINES MESSOBJEKTES**

INHOMOGENEOUS TRANSMISSION LINE FOR DETERMINING THE PERMITTIVITY OF A DEVICE UNDER TEST IN A POSITION-RESOLVED MANNER

LIGNE DE TRANSMISSION NON HOMOGÈNE POUR LA DÉTERMINATION À RÉSOLUTION EN POSITION DE LA PERMITTIVITÉ D'UN OBJET À MESURER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.02.2015 DE 102015201773**

(43) Veröffentlichungstag der Anmeldung:
**13.12.2017 Patentblatt 2017/50**

(73) Patentinhaber: **IHP GmbH-Innovations for High Performance Microelectronics / Leibniz-Institut für innovative Mikroelektronik 15236 Frankfurt / Oder (DE)**

(72) Erfinder:
• **MELIANI, Chafik 12555 Berlin (DE)**
• **GUHA, Subhajit 15230 Frankfurt (Oder) (DE)**

• **JAMAL, Farabi Ibne 10243 Berlin (DE)**

(74) Vertreter: **Eisenführ Speiser Patentanwälte Rechtsanwälte PartGmbB Stralauer Platz 34 10243 Berlin (DE)**

(56) Entgegenhaltungen:
**WO-A1-2012/152618      DE-A1-102006 052 637 US-A1- 2006 247 896**

• **ANDREAS PENIRSCHKE ET AL: "Mikrowellenbasierter Massendurchflusssensor auf Basis von zweidimensionalen linkshändigen Leitungsstrukturen", TECHNISCHES MESSEN TM., Bd. 79, Nr. 3, 1. März 2012 (2012-03-01), Seiten 143-151, XP055256230, DE ISSN: 0171-8096, DOI: 10.1524/teme.2012.0174**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Messträger für eine ortsaufgelöste messtechnische Ermittlung einer von der dielektrischen Permittivität eines auf den Messträger aufzulegenden Messobjektes abhängigen Messgröße. Sie betrifft weiterhin eine Messvorrichtung zur ortsaufgelösten Bestimmung der dielektrischen Permittivität eines Messobjektes sowie ein Verfahren zur ortsaufgelösten Bestimmung der Permittivität eines Messobjektes.

Bei der Charakterisierung von Materialien ist es oftmals notwendig, ortsaufgelöste Messungen durchführen zu können. Nur mit Hilfe von positionsaufgelösten Messungen ist es möglich, Inhomogenitäten oder zufällige Phänomene, beispielsweise in Biomaterialien, zu erfassen.

Hierbei ergibt sich das Problem, dass es oftmals nicht oder nicht zuverlässig möglich ist, einen Sensor in unmittelbarer Nähe des zu charakterisierenden Messobjekts oder eines Teilbereiches des Messobjekts zu positionieren. Dies schränkt die Flexibilität und Sensitivität solcher Messungen ein. Eine ortsaufgelöste Messung kleiner Strukturen wie biologischer Zellen, eine Beobachtung von Zellwachstum, oder eine Diagnose maligner Zellen ist auf diese Weise kaum realisierbar.

Für biologische Messobjekte bestehen alternative Lösungen, die auf Fallen- oder Markierungstechniken (elektrisch oder mechanisch) beruhen. Solche Messtechniken können jedoch die zu ermittelnden Charakteristika der Proben beeinflussen und so Messungen verfälschen oder den mit der Aufbereitung der Messungen verbunden Aufwand erhöhen.

Andere bekannte Lösungen für positionsaufgelöste Messungen beruhen auf der Verwendung einer Vielzahl von Einzelsensoren, wie beispielsweise in US 2006/0247896 beschrieben. Die Verwendung einer Vielzahl von Sensoren führt jedoch zu einer Vielzahl von Ein- und Ausgängen, die in der Messeinrichtung realisiert werden müssen. Dies beschränkt die Möglichkeiten einer Miniaturisierung und damit die maximale Auflösung sowie Realisierungsmöglichkeiten stark.

DE102006052637 A1 beschreibt eine Vorrichtung zur Bestimmung von Mehrphasengemischen, wobei durch Einleitung zumindest eines elektrischen Signals in die Meßstrecke bereichsweise ein elektromagnetisches Feld in das Medium einkoppelbar und/oder auskoppelbar ist, wobei die Meßstrecke zumindest eine Leitungsanordnung mit zumindest zwei entlang der Meßstrecke angeordneten Elementarzellen, die jeweils zumindest eine elektrische Strecke von zumindest einem Eingang zu zumindest einem Ausgang umfassen, aufweist.

Nachfolgend werden Ausführungsformen der vorliegenden Erfindung beschrieben.

Gemäß einem ersten Aspekt der Erfindung wird ein Messträger für eine ortsaufgelöste messtechnische Ermittlung einer von der dielektrischen Permittivität eines auf den Messträger aufzulegenden Messobjektes abhängigen Messgröße, umfassend

- eine Auflage mit einer Messauflagefläche, auf die das Messobjekt aufgelegt werden kann,
- eine die Messauflagefläche ganz oder teilweise bildende Mess-Übertragungsleitung des Messträgers, die zur Übertragung eines einkoppelbaren elektromagnetischen Hochfrequenz-Messsignals als eine elektrische Reihenschaltung einer Vielzahl Übertragungsleitungszellen ausgebildet ist und damit die Messauflagefläche zellenförmig strukturiert, wobei
- jede der Übertragungsleitungszellen der Mess-Übertragungsleitung für sich betrachtet

in einem messobjektfreien Zustand bezüglich des Hochfrequenz-Messsignals eine zellenindividuelle Ausbreitungskonstante aufweist, die von den jeweiligen zellenindividuellen Ausbreitungskonstanten der anderen Übertragungsleitungszellen verschieden ist, und

ausgebildet ist, in einem Mess-Zustand bei Aufliegen des Messobjekts auf der Übertragungsleitungszelle eine im Vergleich mit dem messobjektfreien Zustand verschiedene zellenindividuelle Ausbreitungskonstante anzunehmen, derart, dass

- eine im Mess-Zustand im Vergleich mit dem messobjektfreien Zustand eintretende Änderung der jeweiligen zellenindividuellen Ausbreitungskonstante jeder der Übertragungsleitungszellen eine jeweilige, identifizierbare Änderungskomponente einer Gesamtänderung einer anhand des Hochfrequenz-Messsignals erfassbaren und von der dielektrischen Permittivität der Mess-Übertragungsleitung als Ganzes abhängigen Messgröße bewirkt.

Die vorliegende Erfindung stellt einen Messträger bereit, mit dem positionsaufgelöste Messungen der Permittivität des Messobjektes einfach möglich sind. Die Erfindung beruht auf dem Konzept einer inhomogenen Übertragungsleitung, die Übertragungsleitungsabschnitte mit unterschiedlichen Ausbreitungskonstanten für elektromagnetische Wellen aufweist. Die Ausbreitung eines elektromagnetischen Signals über eine solche inhomogene Übertragungsleitung wird von den Ausbreitungskonstanten der Übertragungsleitungsabschnitten und des umgebenden Materials beeinflusst. Ändert sich mit der Permittivität des Materials in der Umgebung, so beeinflusst dies die Ausbreitung des Signals. Damit ist über die Änderung der Ausbreitungskonstante die Position des Materials bestimmbar und eine Analyse des Materials möglich. Die Position des Materials ergibt sich dabei aus den unterschiedlichen Ausbreitungskonstanten der einzelnen Übertragungsleitungsabschnitte.

Mit dem vorliegend beschriebenen Messträger kann schon mit einer einzelnen Messung anhand einer einzelnen Signalquelle, deren durch sie bereitgestelltes Hochfrequenzsignal durch die Mess-Übertragungsleitung und damit aufgrund ihrer Reihenschaltung durch jede einzelne der Übertragungsleitungszellen geleitet wird, eine die Bestimmung der Permittivität des Messobjekts in allen Übertragungsleitungszellen erlaubende Messgröße erfasst werden. Da die Übertragungsleitungszellen die Messauflagefläche zellenförmig strukturieren, kann anhand einer einzelnen Messung ein ortsaufgelöstes Messdatenfeld ermittelt werden, anhand dessen die Permittivität des Messobjekts in den einzelnen Übertragungsleitungszellen bestimmbar ist.

Der Messträger ist ein in manchen Ausführungsformen auch unabhängig von einer zugehörigen Messvorrichtung handelbares Bauteil, dessen Struktur die vorstehend beschriebene Messtechnik erst ermöglicht.

Nachfolgend werden Ausführungsbeispiele des erfindungsgemäßen Messträgers beschrieben. Die zusätzlichen Merkmale der Ausführungsbeispiele können miteinander kombiniert werden, um weitere Ausführungsbeispiele zu bilden, es sei denn, sie sind ausdrücklich als Alternativen zueinander beschrieben.

In einer Ausführungsform des Messträgers bilden die Übertragungsleitungszellen jeweils ein LC-Glied. Die zellenindividuelle Ausbreitungskonstante ist durch eine zellenindividuell vorbestimmte Kapazität des jeweiligen LC-Glieds realisiert. Unter einem LC-Glied wird dabei eine aus mindestens einem induktiven und mindestens einem kapazitiven Element bestehende Schaltungsanordnung verstanden. Jede Übertragungsleitungszelle beeinflusst in dieser Ausführungsform mit ihrer individuellen Kapazität ein von ihr gebildetes Flächenelement der Messauflagefläche. Die Induktivitäten der LC-Glieder sind in einer Variante dieser Ausführungsform in allen Übertragungsleitungszellen gleich. Als LC-Glied werden hier auch Übertragungsleitungszellen mit ohmschen Widerstands- oder Ableitungskomponenten bezeichnet, welche bei Verwendung geeigneter Frequenzen für das Hochfrequenzmesssignal für die Zwecke der vorliegend behandelten Messung vernachlässigbar sind.

Je nach Frequenzbereich und Anwendung kann der Messträger in Form einer Platine mit gedruckten Übertragungsleitungsstrukturen (printed circuit board, PCB) realisiert werden oder mit den Verfahren der Halbleitertechnologie monolithisch hergestellt werden.

Verschiedene andere Varianten dieser Ausführungsform sind möglich. In einer Variante des Messträgers dieser Ausführungsform weist eine erste Übertragungsleitungszelle eine erste vorbestimmte Kapazität auf. Die weiteren vorbestimmten Kapazitäten der weiteren Übertragungsleitungszellen sind jeweils als Produkt einer zellenindividuellen Potenz eines bestimmten Faktors und der ersten vorbestimmten Kapazität vorbestimmt. Über den Faktor lassen sich Messauflösung und Messdynamik gezielt beeinflussen. Je größer der Faktor ist, desto größer ist der Dynamikbereich. Der Dynamikbereich entspricht einem Messbereich, der durch die Ausbreitungskonstante bestimmt wird und in dem die Permittivität $\varepsilon$ für die jeweiligen Leitungsübertragungszellen einzeln bestimmt werden kann. In einer anderen Variante variiert ein Dynamikbereich zellenindividuell. Dieser Dynamikbereich kann jedoch relativ, also bezogen auf die jeweilige Kapazität, für alle Kapazitäten gleich sein. Dies sei anhand eines Beispiels erläutert. Für alle Übertragungsleitungszellen kann der Dynamikbereich beispielsweise den Betrag der Kapazität der jeweiligen Übertragungsleitungszellen umfassen. Für eine bestimmte Änderung der Permittivität ergibt sich ein Dynamikbereich (der Kapazitätsänderung) von $\dfrac{C}{2}$ bis $\dfrac{3}{2}C$, wobei C die jeweilige Kapazität des betreffenden Übertragungsleitungszelle bezeichnet. Für eine erste Übertragungsleitungszelle mit einer Kapazität $C = C_0$ ergibt sich ein Dynamikbereich von $\dfrac{C_0}{2}$ bis $\dfrac{3C_0}{2}$. Für einen zweiten Übertragungsleitungsabschnitt mit einer Kapazität $C = A \cdot C_0$ ergibt sich der Dynamikbereich $\dfrac{A \cdot C_0}{2}$ bis $\dfrac{3A \cdot C_0}{2}$, und für einen dritten Übertragungsleitungsabschnitt mit der Kapazität $C = A^2 \cdot C_0$ von $\dfrac{A^2 \cdot C_0}{2}$ bis $\dfrac{3A^2 \cdot C_0}{2}$.

Eine auf einer Übertragungsleitungszelle der Übertragungsleitung angeordnete Materialprobe führt zu einem Phasenunterschied des übertragenen Signals im Dynamikbereich der jeweiligen Übertragungsleitungszelle. Für das obige Beispiel ergeben sich daher folgende Phasenunterschiede: Liegt ein Messobjekt auf dem ersten Übertragungsleitungsabschnitt ($C = C_0$) auf, so verursacht dieses einen Phasenunterschied im Bereich von $\dfrac{1}{\sqrt{\dfrac{C_0}{2} \cdot L_0}}$ bis $\dfrac{1}{\sqrt{\dfrac{3C_0}{2} \cdot L_0}}$, wobei $L_0$ die Induktivität des Übertragungsleitungsabschnittes ist. Im vorliegenden Beispiel wird die Induktivität als gleich für alle Übertragungsleitungsabschnitte angenommen. Liegt das Messobjekt auf dem zweiten Übertragungsleitungsabschnitt ($C = A \cdot C_0$) auf, so ergibt sich ein Phasenunterschied im Bereich von $\dfrac{1}{\sqrt{\dfrac{AC_0}{2} \cdot L_0}}$ bis $\dfrac{1}{\sqrt{\dfrac{3AC_0}{2} \cdot L_0}}$

. Liegt das Messobjekt auf dem dritten Übertragungsleitungsabschnitt ($C = A^2 \cdot C_0$) auf, so ergibt sich ein Phasenunterschied im Bereich von $\dfrac{1}{\sqrt{\dfrac{A^2 C_0}{2} \cdot L_0}}$ bis $\dfrac{1}{\sqrt{\dfrac{3 A^2 C_0}{2} \cdot L_0}}$. Somit ist über den Bereich, in dem der Phasenunterschied festgestellt wird, eine Bestimmung des Übertragungsleitungsabschnittes, auf dem sich die Messprobe befindet, möglich. Die gemessenen Phasenunterschiede lassen sich den einzelnen Übertragungsleitungsabschnitten zuordnen.

In einer ersten Gruppe von Ausführungsbeispielen verfügt der Messträger über zwei Anschlüsse, von denen einer einen Eingangsport für das Einkoppeln des Hochfrequenz-Messsignals und der andere einen Ausgangsport für das Ausgeben des durch die Mess-Übertragungsleitung übertragenen Hochfrequenz-Messsignals an die Messvorrichtung bildet, sodass eine Transmissionsmessung möglich ist. Alternativ oder zusätzlich dazu ist eine andere Gruppe von Ausführungsformen für die Durchführung einer Reflexionsmessung eingerichtet, bei der ein Eingangsport und ein Ausgangsport des Messträgers von demselben Anschluss gebildet werden.

Die Induktivitäten der Übertragungsleitungsabschnitte sind in einer Ausführungsform des Messträgers für alle Übertragungsleitungsabschnitte gleich. Damit unterscheiden sich die Übertragungsleitungsabschnitte lediglich durch die unterschiedlichen Kapazitäten. In anderen Ausführungsformen, bei denen die Übertragungsleitungszellen jeweils ein LC-Glied bilden, ist die zellenindividuelle Ausbreitungskonstante durch eine zellenindividuell vorbestimmte Induktivität des jeweiligen LC-Glieds realisiert. In manchen solcher Ausführungsformen ist die Kapazität aller Übertragungsleitungszellen identisch. Auch hier ist die zellenindividuelle Ausgestaltung in einem Beispiel so realisiert, dass eine erste Übertragungsleitungszelle eine erste vorbestimmte Induktivität aufweist und weitere vorbestimmte Induktivitäten der weiteren Übertragungsleitungszellen als Produkt einer zellenindividuellen Potenz eines bestimmten Faktors und der ersten vorbestimmten Induktivität vorbestimmt sind. Weisen die Übertragungsleitungsabschnitte unterschiedliche Induktivitäten auf, so wird die Impedanzanpassung der Leitung verbessert und die Messung vereinfacht. Zudem können Messabschnitte aus mehreren Übertragungsleitungszellen gebildet werden.

In einer weiteren Ausführungsform des Messträgers bildet mindestens eine Übertragungsleitungszelle einen linkshändigen Abschnitt der Übertragungsleitung. Eine linkshändige Übertragungsleitung oder ein linkshändiger Abschnitt davon hat eine negative Permittivität und somit einer negative Ausbreitungskonstante für das Hochfrequenzmesssignal. Linkshändige Strukturen haben bei vergleichbaren Dimensionen eine vergleichbare oder sogar höhere Sensitivität als rechtshändige Strukturen. Beispielsweise kann eine Übertragungsleitung eine erste Übertragungsleitungszelle, die einen gewöhnlichen, also rechtshändigen Leitungsabschnitt bildet, und eine zweite Übertragungsleitungszelle mit einer linkshändigen Struktur aufweisen.

Da die Ausbreitungskonstanten für links- und rechtshändige Strukturen typischerweise eine unterschiedliche Abhängigkeit der Permittivität von der Frequenz aufweisen, können über die Erfassung von Änderungen der Ausbreitungskonstanten in unterschiedlichen Frequenzbereichen des Hochfrequenzmesssignals sowohl unterschiedliche Permittivitäten als auch die Orte dieser unterschiedlichen Permittivitäten bestimmt werden.

Insbesondere ist es vorteilhaft, wenn die Mess-Übertragungsleitung in ihrer Gesamtheit als Reihenschaltung linkshändiger und rechtshändiger Abschnitte in Form der jeweiligen Übertragungsleitungszellen im messobjektfreien Zustand für das Hochfrequenz-Messsignal eine Ausbreitungskonstante von Null aufweist. In diesem Ausführungsbeispiel führt ein auf dem Übertragungsleitungsabschnitt angeordnetes Material zu einer Änderung der Ausbreitungskonstante entweder in positive oder in negative Richtung.

In einer weiteren Ausführungsform des Messträgers ist die Messauflagefläche zusätzlich von einer zweiten Mess-Übertragungsleitung in elektrischer Parallelschaltung zur vorstehend beschriebenen Mess-Übertragungsleitung gebildet. Jede Übertragungsleitungszelle der ersten Mess-Übertragungsleitung ist mittels einer jeweiligen aktiven elektronischen Komponente, vorzugsweise mittels eines Transistors, mit einer zugeordneten Übertragungsleitungszelle der zweiten Mess-Übertragungsleitung gekoppelt. Bei dieser Ausführungsform weist der Messträger also eine parallelgeschaltete zweite Mess-Übertragungsleitung in der Auflage auf, die aktiv an die (erste) Mess-Übertragungsleitung gekoppelt ist. In dieser Ausführungsform der Erfindung wird Zelle für Zelle eine Teilamplitude des Hochfrequenzmesssignals, das über die erste Mess-Übertragungsleitung übertragen wird, in die zweite Übertragungsleitung eingekoppelt. Ein auf einem der Übertragungsleitungsabschnitte der ersten oder der zweiten Übertragungsleitung angeordnetes Messobjekt ändert den Kopplungskoeffizienten für die jeweiligen Übertragungsleitungszellen und damit die Ausbreitungskonstante und die Streumatrixparameter, wobei die Streumatrixparameter auch als S-Parametermatrix bezeichnet werden, wie sie anhand des am Ausgangsport anliegenden Hochfrequenzsignals ermittelbar sind. Somit ist auch mit dieser Ausführungsform eine Bestimmung des Ortes sowie der Permittivität eines Messobjektes möglich.

Erfindungsgemäß bildet die Mess-Übertragungsleitung wie erwähnt zumindest einen Teil der Messauflagefläche. Dies kann beispielsweise in Form eines direkten, also unmittelbaren Kontakts zwischen der Mess-Übertragungsleitung, also den jeweiligen Übertragungsleitungszellen, und einem aufzulegenden Messobjekt realisiert sein. In einer anderen Ausführungsform des Messträgers hat die Übertragungsleitung eine Schutzschicht, zu der das bei einer Messung aufgelegte Messobjekt unmittelbaren Kontakt hat. Eine solche Schutzschicht ist für die Zwecke dieser Ausführungsform als Teil der Mess-Übertragungsleitung zu verstehen. Mit dieser Ausführungsform ist es möglich, die Übertragungsleitung gegen schädliche Einflüsse der Umgebung oder des Messobjektes, wie beispielsweise Korrosion zu schützen.

Gemäß einem zweiten Aspekt betrifft die Erfindung eine Messvorrichtung zur ortsaufgelösten Bestimmung der dielektrischen Permittivität eines Messobjektes, umfassend

- eine Steuerungseinrichtung, die ausgebildet ist, ein vorbestimmtes Hochfrequenz-Messsignal bereitzustellen;

- eine Ausgangs-Schnittstelle zur Übergabe des Hochfrequenz-Messsignals an Messträger nach einem der Ansprüche 1 bis 9 und eine Eingangsschnittstelle zum Empfang des durch die Mess-Übertragungsleitung des Messträgers übertragenen Hochfrequenz-Messsignals vom Messträger; und

- eine Auswerteeinheit, die ausgebildet ist, anhand des von der Eingangsschnittstelle unter Aufliegen des Messobjekts im Mess-Zustand empfangenen Hochfrequenzsignals eine von der Ausbreitungskonstante der Mess-Übertragungsleitung abhängige Messgröße zu erfassen, die erfasste Messgröße mit einer im messobjektfreien Zustand der Mess-Übertragungsleitung ermittelten Probe der Messgröße zu vergleichen und anhand des Vergleichsergebnisses und anhand vorgespeicherter zellenindividueller Parameter zellenindividuelle Änderungskomponenten der Ausbreitungskonstante zu bestimmen und daraus zellenindividuelle Permittivitätswerte des Messobjekts zu ermitteln.

Unter dem Begriff Probe einer Messgröße werden unterschiedliche Ausführungsformen verstanden. Beispielsweise kann die Probe der Messgröße Kalibrierwerte beinhalten oder Signalmuster.

Die Messvorrichtung teilt die Vorteile des Messträgers des ersten Aspekts der Erfindung. Einige ihrer Ausführungsformen werden nachfolgend beschrieben.

In einer bevorzugten Form der Erfindung ist die Steuerungseinrichtung ausgebildet, eine Signalerzeugung des Hochfrequenzsignals als Folge von Signalen mit mehreren unterschiedlichen Frequenzen zu steuern.

Die vorgespeicherten zellenindividuellen Parameter sind geeignet, eine zellenindividuelle Ausbreitungskonstante der jeweiligen Übertragungsleitungszelle im messobjektfreien Zustand rechnerisch zu bestimmen. Sie werden entweder in der Messvorrichtung, beispielsweise in der Auswerteeinheit selbst hinterlegt oder können im Rahmen einer zusätzlichen Messung erfasst werden.

Die Messvorrichtung ist in einer Variante mit dem Messträger monolithisch integriert.

Gemäß einem dritten Aspekt betrifft die Erfindung ein Verfahren zur ortsaufgelösten Bestimmung der Permittivität eines Messobjektes, umfassend die Schritte:

- Bereitstellen eines Messträgers gemäß dem ersten Aspekt der Erfindung oder einer seiner offenbarten Ausführungsformen;

- Auflegen des Messobjektes auf die Auflage des Messträgers;

- Bereitstellen eines Hochfrequenzmesssignals an der Mess-Übertragungsleitung des Messträgers;

- Erfassen des durch die Mess-Übertragungsleitung übertragenen Hochfrequenzmesssignals, beispielsweise an einem Ausgangsport (Transmission) oder an einem für das Bereitstellen des Hochfrequenzmesssignals genutzten Eingangsport (Reflexion) des Messträgers;

- Ermitteln einer von der Ausbreitungskonstante der Mess-Übertragungsleitung abhängige Messgröße anhand des erfassten Hochfrequenzmesssignals;

- Vergleichen der ermittelten Messgröße mit einer im messobjektfreien Zustand der Mess-Übertragungsleitung ermittelten Probe der Messgröße;

- Zellenindividuelles Ermitteln von Änderungskomponenten der Ausbreitungskonstante anhand des Vergleichsergebnisses und anhand vorgespeicherter zellenindividueller Parameter;

- Bestimmen zellenindividueller Permittivitätswerte des Messobjekts anhand der ermittelten zellenindividuellen Änderungskomponenten.

In einer bevorzugten Ausführungsform der Erfindung wird das Verfahren mehrfach bei unterschiedlichen Frequenzen des Hochfrequenzmesssignals durchgeführt.

Geeignete Messverfahren im Rahmen der Erfindung sind beispielsweise S-Parametermessungen bzw. Transmissionsmessungen oder Reflexionsmessungen oder auch Gain- bzw. Verlustmessungen der Leitungen. Über die gemessenen Verluste können die Permittivitätswerte der auf den einzelnen Übertragungsleitungsabschnitten angeordneten Teile des Messobjektes bestimmt werden.

Die Messvorrichtung sowie das Verfahren zur Messung der Permittivität teilen die Vorteile des Messträgers gemäß dem ersten Aspekt der Erfindung und haben einander entsprechende Ausführungsbeispiele. Ausführungsformen der Erfindung sind auch in den Ansprüchen beschrieben.

Weitere Ausführungsbeispiele des erfindungsgemäßen Messträgers sowie der Messvorrichtung und des Verfahrens zur Messung der Permittivität werden nachfolgend anhand der Figuren erläutert. Dabei zeigt

Fig. 1     schematisch ein Schaltbild einer Ausführungsform eines Messträgers gemäß dem ersten Aspekt der Erfindung,

Fig. 2     schematisch ein Schaltbild einer weiteren Ausführungsform eines Messträgers gemäß dem ersten Aspekt der Erfindung,

Fig. 3     schematisch ein Schaltbild einer dritten Ausführungsform eines Messträgers gemäß dem ersten Aspekt der Erfindung,

Fig. 4     schematisch eine vierte Ausführungsform eines Messträgers gemäß dem ersten Aspekt der Erfindung,

Fig. 5     schematisch eine Ausführungsform einer Messvorrichtung gemäß dem zweiten Aspekt der Erfindung,

Fig. 6     schematisch eine Ausführungsform eine integrierte Messvorrichtung gemäß dem dritten Aspekt der Erfindung,

Fig. 7     schematisch eine Ausführungsform eines Verfahrens gemäß dem vierten Aspekt der Erfindung.

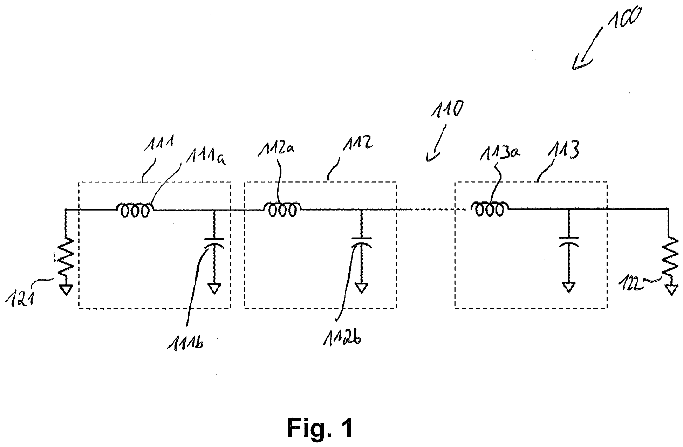

Fig. 1 zeigt schematisch ein Schaltbild einer Ausführungsform eines Messträgers 100 gemäß dem ersten Aspekt der Erfindung.

Der Messträger 100 weist eine Mess-Übertragungsleitung 110 auf, die zur Übertragung eines Hochfrequenzmesssignals dient und die eine Vielzahl elektrisch in Reihe geschalteter Übertragungsleitungszellen aufweist, die hier in Form eines vereinfachten Beispiels von drei Übertragungsleitungszellen 111, 112, 113 repräsentiert sind. Die Gesamtheit der Übertragungsleitungszellen bildet eine für den jeweiligen Anwendungsfall geeignet gestaltete Messauflagefläche, die entsprechend zellulär strukturiert ist. Jede der Übertragungsleitungszellen 111, 112, 113 weist dabei eine zellenindividuelle Ausbreitungskonstante für das Hochfrequenzmesssignal auf, die verschieden ist von den Ausbreitungskonstanten der weiteren Übertragungsleitungszellen. Die Übertragungsleitungszellen 111, 112, 113 weisen jeweils eine Induktivität 111a, 112a, 113a als Teil der Leitung und eine Kapazität 111b, 112b, 113b auf. Die unterschiedlichen Übertragungsleitungszellen 111, 112, 113 weisen im vorliegenden Ausführungsbeispiel unterschiedliche Kapazitäten auf. Somit variieren auch die jeweiligen, für sich betrachteten Ausbreitungskonstanten der unterschiedlichen Übertragungsleitungszellen, die zur Ausbreitungskonstante der Mess-Übertragungsleitung als ganzer beitragen.

Wird auf die Messauflagefläche des Messträgers ein Messobjekt aufgelegt, so gelangt es in Kontakt mit den Übertragungsleitungszellen und beeinflusst mit seiner dielektrischen Permittivität am jeweiligen Ort die zellenindividuellen Ausbreitungskonstanten der unterschiedlichen, vom Messobjekt belegten Übertragungsleitungszellen und führt somit zu zellenindividuellen Beiträgen zu Verlusten und Phasenunterschieden des übertragenen Signals. Es werden somit die Oszillationscharakteristiken geändert.

Jede Übertragungsleitungszelle 111, 112, 113 verfügt im vorliegenden Ausführungsbeispiel über einen Dynamikbereich, der um die Kapazität des jeweiligen Übertragungsleitungsabschnittes variiert und in dem die Permittivität ε bestimmt werden kann..

Die Kapazitäten der Übertragungsleitungszellen sind im vorliegenden Beispiel derart ausgewählt, dass ein erster Übertragungsleitungsabschnitt 111 eine erste Kapazität aufweist und die weiteren Kapazitäten der weiteren Übertragungsleitungszellen 112, 113 einem Produkt eines Faktors A bzw. seiner aufsteigenden Potenzen mit der ersten Kapazität entsprechen. Abhängig im Wesentlichen von der Kapazität und Induktivität der jeweiligen Übertragungsleitungszelle trägt diese nur zu einem zellenindividuellen Anteil zu Verlusten und Phasenunterschieden des Hochfrequenzmesssignals bei der Übertragung durch die Mess-Übertragungsleitung bei. Diese Anteile sind so ausgelegt, dass ein eindeutiger Rückschluss vom gemessenen Gesamt-Phasenunterschied oder Gesamt-Verlust auf die daran beteiligten Übertragungsleitungszellen möglich ist. Auf diese Weise kann anhand der gemessenen Änderung auf die dafür ursächliche Permittivität des Messobjekts am Ort der jeweiligen Übertragungsleitungszelle zurückgeschlossen werden.

In einer weiteren Ausführungsform der Erfindung, die hier nicht dargestellt ist, verfügen die Übertragungsleitungszellen 111, 112, 113 zusätzlich zu unterschiedlichen Kapazitäten auch über unterschiedliche Induktivitäten.

Die Mess-Übertragungsleitung weist im vorliegenden Ausführungsbeispiel zwei Anschlüsse 121, 122 für eine Messvorrichtung auf, die einen Eingangs- und einen Ausgangsport des Messträgers bilden. Damit ergeben sich zwei Möglichkeiten der Messung, zum einen Transmissionsmessung, bei der beide Anschlüsse 121, 122 verwendet werden und die Transmission eines Signals über die Übertragungsleitung 110 gemessen wird.

Darüber hinaus ermöglicht der Messträger 100 aber auch eine Messung an nur einem der Anschlüsse 121, der also Eingangs- und Ausgangsport zugleich bildet. Hierzu wird der zweite Anschluss 122 mit einem Kurzschluss oder Leerlauf terminiert, sodass eine volle Reflektion am Anschluss 122 stattfindet. Damit verdoppelt sich die Messstrecke. Die Messung erfolgt analog einer Messung bei Transmission lediglich mit dem Unterschied, dass der gemessene Phasenverlauf und die Verluste der Leitung der doppelten Strecke entsprechen. Bei einer Messung an nur einem Anschluss können zusätzlich bestimmte Anschlusswiderstände statt des Kurzschlusses oder Leerlaufs in den Aufbau integriert werden. Mithilfe dieser zusätzlichen Widerstände können die Messbedingungen beeinflusst und eine höhere Messgenauigkeit erreicht werden.

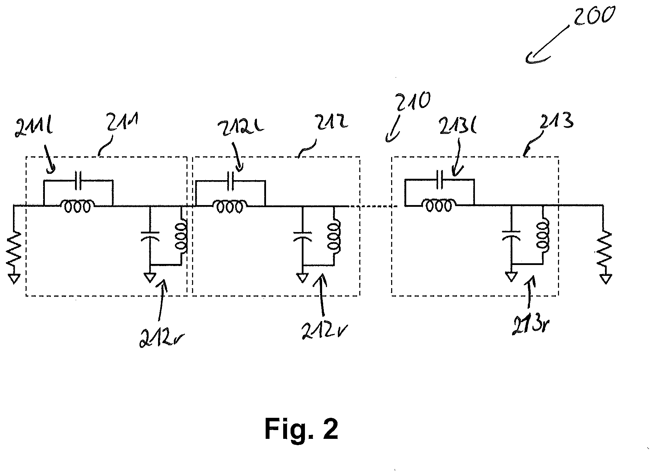

Fig. 2 zeigt schematisch ein Schaltbild einer weiteren Ausführungsform eines Messträgers 200 gemäß dem ersten Aspekt der Erfindung. Im Unterschied zum in Fig. 1 gezeigten Messträger sind die Übertragungsleitungszellen 211, 212, 213 der Mess-Übertragungsleitung 210 in der gezeigten Ausführungsform linkshändige Strukturen 211l, 212l, 213l in Kombination mit rechtshändigen Strukturen 211r, 212r, 213r. Die linkshändigen und rechtshändigen Anteile der jeweiligen Übertragungsleitungsabschnitte sind dabei derart ausgelegt, dass die Ausbreitungskonstante insgesamt gleich 0 ist. Diese ergibt sich aus den negativen zellenindividuellen Ausbreitungskonstanten der linkshändigen Abschnitte in den betreffenden Übertragungsleitungszellen und den positiven zellenindividuellen Ausbreitungskonstanten der rechtshändigen Abschnitte in den anderen betreffenden Übertragungsleitungszellen. Messobjekte, die auf einer jeweiligen der Übertragungsleitungszellen angeordnet sind, ändern somit die jeweilige zellenindividuelle Ausbreitungskonstante hin zum Positiven oder Negativen. Damit ist eine zellenindividuelle und damit ortsaufgelöste Bestimmung einer ortsabhängigen dielektrischen Permittivität des Materials des Messobjekts an den unterschiedlichen belegten Übertragungsleitungszellen 211, 212, 213 möglich. Die Messung an einem Messträger 200, wie in Fig. 2 gezeigt, erfolgt analog den im Hinblick auf Fig. 1 beschriebenen Mess-Varianten.

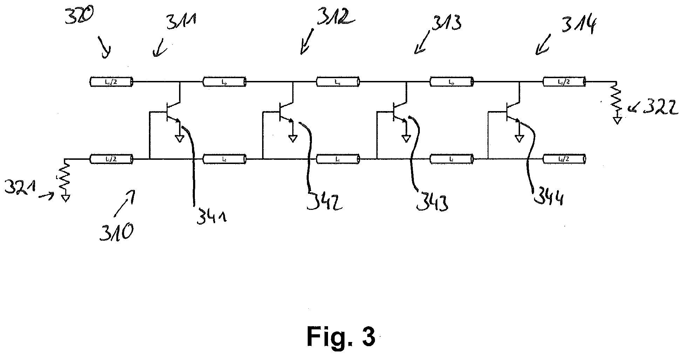

Fig. 3 zeigt schematisch ein Schaltbild einer dritten Ausführungsform eines Messträgers 300 gemäß dem ersten Aspekt der Erfindung. Der Messträger 300 weist zwei Mess-Übertragungsleitungen 310, 320 auf, die elektrisch parallel geschaltet und aktiv aneinander gekoppelt sind. Die Kopplung findet über aktive Komponenten, im gezeigten Ausführungsbeispiel über Bipolartransistoren BJT statt. Die elektromagnetische Welle des Hochfrequenzmesssignals, die sich vom als Eingangsport dienenden Anschluss 321 durch die Mess-Übertragungsleitungen ausbreitet, wird in einer Richtung über die Bipolartransistoren 341, 342, 343, 344 auf ein Signal der zweiten Übertragungsleitung aufgekoppelt. Ein auf einer der Übertragungsleitungszellen 311, 312, 313, 314 angeordnetes Messobjekt ändert den Kopplungskoeffizienten für diese Übertragungsleitungszelle und damit die Ausbreitungskonstante bzw. den Streumatrixparameter.

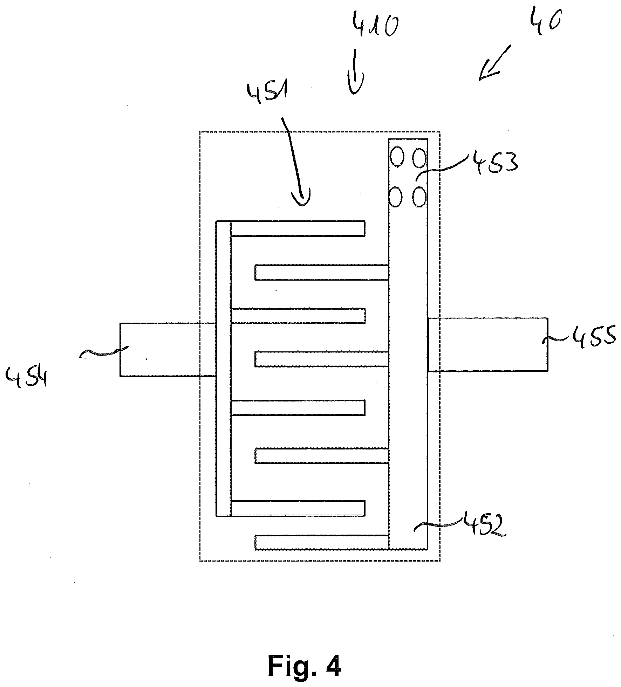

Fig. 4 zeigt einen Ausschnitt einer vierten Ausführungsform eines Messträgers gemäß dem ersten Aspekt der Erfindung. Die hier gezeigte Ausführungsform des Messträgers 400 kann sowohl hybrid, beispielsweise auf einer Leiterplatte, als auch monolithisch, beispielsweise auf einem Chip realisiert werden. Die hybride Realisierung ist für Messträger für Messfrequenzen im Bereich von 1 GHz bis 5 GHz sinnvoll, monolithische Realisierungen empfehlen sich für höhere Frequenzen. Wesentlich geringere Frequenzen als 1 GHz sind hier nicht vorgesehen. Der hier gezeigte Abschnitt einer Übertragungsleitung 410 des Messträgers weist eine Übertragungsleitungszelle auf, die von linkshändige Abschnitten gebildet wird. Dargestellte ineinandergreifende fingerartige Strukturen 451 bilden eine Kapazität. Die Anschlüsse 454 und 455 stellen Elektroden dar. Der Anschluss der Kapazität ist über einen oder mehrere Vias 453 ausgeführt.

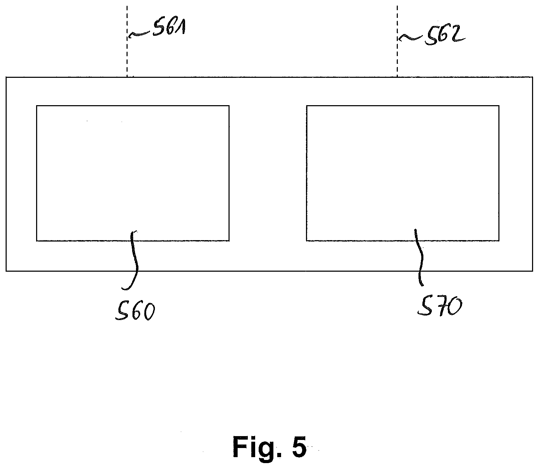

Fig. 5 zeigt schematisch eine Ausführungsform einer Messvorrichtung 500 gemäß dem zweiten Aspekt der Erfindung. Die Messvorrichtung 500 umfasst eine Steuerungseinrichtung 560 sowie eine Auswerteeinheit 570.

Die Steuerungseinrichtung 560 ist ausgebildet, eine Signalerzeugung zu veranlassen, also ein vorbestimmtes Hochfrequenz-Messsignal bereitzustellen.

Darüberhinaus weist die Messvorrichtung eine Ausgangs-Schnittstelle 561 zur Übergabe des Hochfrequenz-Messsignals an den Eingangsport eines Messträgers wie beispielswiese in Fig. 1 dargestellt und eine Eingangsschnittstelle 562 zum Empfang des durch die Mess-Übertragungsleitung des Messträgers übertragenen Hochfrequenz-Messsignals vom Ausgangsport des Messträgers auf.

Die Auswerteeinheit 570 ist ausgebildet, anhand des von der Eingangsschnittstelle unter Aufliegen des Messobjekts im Mess-Zustand empfangenen Hochfrequenzsignals eine von der Ausbreitungskonstante der Mess-Übertragungsleitung abhängige Messgröße zu erfassen, die erfasste Messgröße mit einem im messobjektfreien Zustand der Mess-Übertragungsleitung ermittelten Probe der Messgröße zu vergleichen und anhand des Vergleichsergebnisses und anhand vorgespeicherter zellenindividueller Parameter zellenindividuelle Änderungskomponenten der Ausbreitungskonstante zu bestimmen und daraus zellenindividuelle Permittivitätswerte des Messobjekts zu ermitteln. Die zellenindividuellen Parameter sind Kapazität, Induktivität oder Kopplungskoeffizient des von der jeweiligen Übertragungsleitungszelle gebildeten Übertragungsleitungsabschnittes. Diese Kenngrößen erlauben Rückschlüsse auf das Ausbreitungsverhalten des Hochfrequenzmesssignals in der jeweiligen Übertragungsleitungszelle und über die Änderung des Ausbreitungsverhaltens beim Anordnen eines Messobjektes auf einer Übertragungsleitung - somit auch Rückschlüsse auf die Position und die Permittivität des Messobjektes am Ort der belegten Übertragungsleitungszellen, nämlich über die Änderung im Ausbreitungsverhalten.

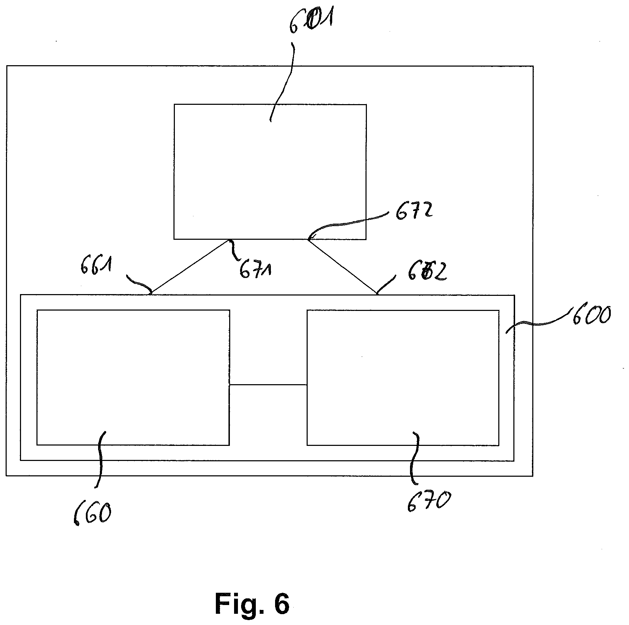

Fig. 6 zeigt schematisch eine Messvorrichtung 600, die mit dem Messträger 601 monolithisch integriert ist. Die integrierte Messvorrichtung 600 dabei wie bereits die in Fig. 5 beschriebene Messvorrichtung eine Steuerungseinrichtung 660, eine Auswerteeinheit 670 sowie eine Ausgangsschnittstelle 661 und eine Eingangsschnittstelle 662. Die Ausgangsschnittstelle 661 ist dabei mit einem Eingangsport 671 des Messträgers 601 und die Eingangsschnittstelle 662 mit einem Ausgangsport 672 des Messträgers 601 verbunden. Eine monolithische Integration der Messvorrichtung 600 mit dem Messträger 601 ermöglicht eine kompakte Bauweise einer Messeinrichtung zur Bestimmung der Permittivität.

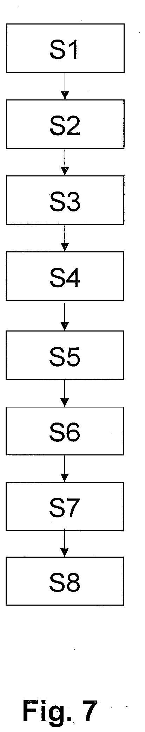

Fig. 7 zeigt schematisch ein Verfahren zur positionsaufgelösten Messung von Permittivitätswerten eines Objektes gemäß dem dritten Aspekt der Erfindung. Zunächst wird im Schritt S1 ein Messträger gemäß dem ersten Aspekt der Erfindung bereitgestellt, wie er beispielhaft in Fig. 1 gezeigt ist. In Schritt S2 wird dann das Messobjekt auf die Auflage des Messträgers aufgelegt. In Schritt S3 wird ein elektromagnetisches Hochfrequenzmesssignals am Eingangsport der Mess-Übertragungsleitung des Messträgers bereitgestellt. In Schritt S4 wird dann das durch die Mess-Übertragungsleitung übertragene Hochfrequenzmesssignal am Ausgangsport des Messträgers empfangen und eine von der Ausbreitungskonstante der Mess-Übertragungsleitung abhängige Messgröße anhand des erfassten Hochfrequenzmesssignals ermittelt (Schritt S5). Anschließend wird die ermittelte Messgröße mit einer im messobjektfreien Zustand der Mess-Übertragungsleitung ermittelten Probe der Messgröße verglichen (Schritt S6). In Schritt S7 werden zellenindividuell Änderungskomponenten der Ausbreitungskonstante anhand des Vergleichsergebnisses und anhand vorgespeicherter zellenindividueller Parameter ermittelt und in Schritt S8 zellenindividuelle Permittivitätswerte des Messobjekts anhand der ermittelten zellenindividuellen Änderungskomponenten bestimmt.

**Patentansprüche**

1. Messträger (100) für eine ortsaufgelöste messtechnische Ermittlung einer von der dielektrischen Permittivität eines auf den Messträger (100) aufzulegenden Messobjektes abhängigen Messgröße, umfassend

    - eine Auflage mit einer Messauflagefläche, auf die das Messobjekt aufgelegt werden kann,
    - eine die Messauflagefläche ganz oder teilweise bildende Mess-Übertragungsleitung (110), die zur Übertragung eines einkoppelbaren elektromagnetischen Hochfrequenz-Messsignals als eine elektrische Reihenschaltung einer Vielzahl Übertragungsleitungszellen (111, 112, 113) ausgebildet ist und damit die Messauflagefläche zellenförmig strukturiert, wobei
- jede der Übertragungsleitungszellen (111, 112, 113) der Mess-Übertragungsleitung (110) für sich betrachtet

    in einem messobjektfreien Zustand bezüglich des Hochfrequenz-Messsignals eine zellenindividuelle Ausbreitungskonstante aufweist, die von den jeweiligen zellenindividuellen Ausbreitungskonstanten der anderen Übertragungsleitungszellen (111, 112, 113) verschieden ist, und
    ausgebildet ist, in einem Mess-Zustand bei Aufliegen des Messobjekts auf der Übertragungsleitungszelle (111, 112, 113) eine im Vergleich mit dem messobjektfreien Zustand verschiedene zellenindividuelle Ausbreitungskonstante anzunehmen, derart, dass

    - eine im Mess-Zustand im Vergleich mit dem messobjektfreien Zustand eintretende Änderung der jeweiligen zellenindividuellen Ausbreitungskonstante jeder der Übertragungsleitungszellen (111, 112, 113) eine jeweilige, identifizierbare Änderungskomponente einer Gesamtänderung einer anhand des Hochfrequenz-Messsignals erfassbaren und von der dielektrischen Permittivität der Mess-Übertragungsleitung (110) als Ganzes abhängigen Messgröße bewirkt.

2. Messträger (100) nach Anspruch 1, bei dem die Übertragungsleitungszellen (111, 112, 113) jeweils ein LC-Glied bilden, und bei dem die zellenindividuelle Ausbreitungskonstante durch eine zellenindividuell vorbestimmte Kapazität (111b, 112b, 113b) des jeweiligen LC-Glieds realisiert ist.

3. Messträger (100) nach Anspruch 2, bei dem eine erste Übertragungsleitungszelle (111) eine erste vorbestimmte Kapazität (111b) aufweist und weitere vorbestimmte Kapazitäten (112b, 113b) der weiteren Übertragungsleitungszellen (112, 113) als Produkt einer zellenindividuellen Potenz eines bestimmten Faktors und der ersten vorbestimmten Kapazität (111b) vorbestimmt sind.

4. Messträger (100) nach einem der vorstehenden Ansprüche, bei dem die Übertragungsleitungszellen (111, 112, 113) jeweils ein LC-Glied bilden, und bei dem die zellenindividuelle Ausbreitungskonstante durch eine zellenindividuell vorbestimmte Induktivität (111a, 112a, 113a) des jeweiligen LC-Glieds realisiert ist.

5. Messträger (100) nach Anspruch 4, bei dem eine erste Übertragungsleitungszelle (111) eine erste vorbestimmte Induktivität (111a) aufweist und weitere vorbestimmte Induktivitäten (112a, 113a) der weiteren Übertragungsleitungszellen (112, 113) als Produkt einer zellenindividuellen Potenz eines bestimmten Faktors und der ersten vorbestimmten Induktivität (111a) vorbestimmt sind.

6. Messträger (100) nach einem der vorstehenden Ansprüche, bei dem mindestens eine Übertragungsleitungszelle (111, 112, 113) einen linkshändigen Abschnitt der Mess-Übertragungsleitung (110) bildet.

7. Messträger (100) nach Anspruch 6, bei dem die Mess-Übertragungsleitung (110) in ihrer Gesamtheit als Reihenschaltung linkshändiger und rechtshändiger Abschnitte in Form der jeweiligen Übertragungsleitungszellen (111, 112, 113) im messobjektfreien Zustand für das Hochfrequenz-Messsignal eine Ausbreitungskonstante von Null aufweist.

8. Messträger (100) nach einem der vorstehenden Ansprüche, bei dem die Messauflagefläche zusätzlich von einer zweiten Mess-Übertragungsleitung (210) in elektrischer Parallelschaltung zur Mess-Übertragungsleitung (110) gebildet ist, und bei dem jede Übertragungsleitungszelle (111, 112, 113) der ersten Mess-Übertragungsleitung (110) mittels einer jeweiligen aktiven elektronischen Komponente, insbesondere mittels eines Transistors, mit einer zugeordneten Übertragungsleitungszelle (211, 212, 213) der zweiten Mess-Übertragungsleitung (210) gekoppelt ist.

9. Messträger (100) nach einem der vorstehenden Ansprüche, bei dem die Mess-Übertragungsleitung (110) eine Schutzschicht aufweist, die ausgebildet ist, das Material der Mess-Übertragungsleitung (110) vor einer mechanischen oder chemischen Wechselwirkung mit dem aufzulegenden Messobjekt zu schützen.

10. Messvorrichtung (600) zur ortsaufgelösten Bestimmung der dielektrischen Permittivität eines Messobjektes, umfassend

- eine Steuerungseinrichtung (660), die ausgebildet ist, ein vorbestimmtes Hochfrequenz-Messsignal bereitzustellen;
- eine Ausgangs-Schnittstelle (661) zur Übergabe des Hochfrequenz-Messsignals an einen Messträger (100) nach einem der Ansprüche 1 bis 9 und eine Eingangsschnittstelle (662) zum Empfang des durch die Mess-Übertragungsleitung (110) des Messträgers (100) übertragenen Hochfrequenz-Messsignals vom Messträger (100); und
- eine Auswerteeinheit (570), die ausgebildet ist, anhand des von der Eingangsschnittstelle (662) unter Aufliegen des Messobjekts im Mess-Zustand empfangenen Hochfrequenzsignals eine von der Ausbreitungskonstante der Mess-Übertragungsleitung (110) abhängige Messgröße zu erfassen, die erfasste Messgröße mit einer im messobjektfreien Zustand der Mess-Übertragungsleitung (110) ermittelten Probe der Messgröße zu vergleichen und anhand des Vergleichsergebnisses und anhand vorgespeicherter zellenindividueller Parameter zellenindividuelle Änderungskomponenten der Ausbreitungskonstante zu bestimmen und daraus zellenindividuelle Permittivitätswerte des Messobjekts zu ermitteln

wobei die vorgespeicherten zellenindividuellen Parameter geeignet sind, eine zellenindividuelle Ausbreitungskonstante der jeweiligen Übertragungsleitungszelle (111, 112, 113) im messobjektfreien Zustand rechnerisch zu bestimmen und wobei die zellenindividuelle Ausbreitungskonstante der jeweiligen Übertragungsleitungszelle (111, 112, 113) von den jeweiligen zellenindividuellen Ausbreitungskonstanten der anderen Übertragungsleitungszellen (111, 112, 113) verschieden ist.

11. Messvorrichtung (600) nach Anspruch 10, bei der die Steuerungseinrichtung (660) ausgebildet ist, das Hochfrequenz-Messsignal als Folge mehrerer Signale mit mehreren unterschiedlichen Frequenzen bereitzustellen.

12. Messvorrichtung (600) nach einem der Ansprüche 10 oder 11, die mit dem Messträger (100) monolithisch integriert ist.

13. Verfahren zur ortsaufgelösten Bestimmung der Permittivität eines Messobjektes, umfassend die Schritte:

- Bereitstellen eines Messträgers (100) gemäß einem der Ansprüche 1 bis 9;
- Auflegen des Messobjektes auf die Auflage des Messträgers (100);
- Bereitstellen eines elektromagnetischen Hochfrequenzmesssignals an der Mess-Übertragungsleitung (110) des Messträgers (100);
- Erfassen des durch die Mess-Übertragungsleitung (110) übertragenen Hochfrequenzmesssignals;
- Ermitteln einer von der Ausbreitungskonstante der Mess-Übertragungsleitung (110) abhängige Messgröße anhand des erfassten Hochfrequenzmesssignals;
- Vergleichen der ermittelten Messgröße mit einer im messobjektfreien Zustand der Mess-Übertragungsleitung (110) ermittelten Probe der Messgröße;
- Zellenindividuelles Ermitteln von Änderungskomponenten der Ausbreitungskonstante anhand des Vergleichsergebnisses und anhand vorgespeicherter zellenindividueller Parameter;
- Bestimmen zellenindividueller Permittivitätswerte des Messobjekts anhand der ermittelten zellenindividuellen Änderungskomponenten.

**Claims**

1.  Measurement carrier (100) for a location resolved metrological determination of a measurement variable dependent on the dielectric permittivity of a measurement object which is to be laid on the measurement carrier (100), comprising

    - a support having a measurement support surface onto which the measurement object can be laid,
    - a measurement transmission line (110) forming the measurement support surface completely or partially, which is designed for the transmission of an electromagnetic high-frequency measurement signal which can be coupled-in as an electrical series circuit of a multiplicity of transmission line cells (111, 112, 113) and thereby structures the measurement support surface in a cellular manner, wherein
    - each of the transmission line cells (111, 112, 113) of the measurement transmission line (110) considered per se

      in a measurement object-free state has, with regard to the high-frequency measurement signal, a propagation constant which is individual to each cell, which is different from the respective propagation constants, individual for each cell, of the other transmission line cells (111, 112, 113), and is designed, in a measuring state, when the measurement object is laid onto the transmission line cell (111, 112, 113) to receive a propagation constant, individual for each cell, which is different in comparison with a measurement object-free state, such that

    - a change, occurring in the measuring state, in comparison with the measurement object-free state, in the respective propagation constant, individual for each cell, of each of the transmission line cells (111, 112, 113), effects a respective identifiable change component of an entire change in a measurement variable detectable on the basis of the high-frequency measurement signal and dependent on the dielectric permittivity of the measurement transmission line (110) as a whole.

2.  Measurement carrier (100) according to claim 1, in which the transmission line cells (111, 112, 113) form in each case an LC member, and in which the propagation constant, individual for each cell, is realized by a predetermined capacity (111b, 112b, 113b), individual for each cell, of the respective LC member.

3.  Measurement carrier (100) according to claim 2, in which a first transmission line cell (111) has a first predetermined capacity (111b) and further predetermined capacities (112b, 113b) of the further transmission line cells (112, 113) are predetermined as a product of an exponentiation, individual to each cell, of a specific factor and the first predetermined capacity (111b).

4.  Measurement carrier (100) according to any of the preceding claims, in which the transmission line cells (111, 112, 113) each form an LC member, and in which the propagation constant, individual to each cell, is realized by a predetermined inductance (111a, 112a, 113a), individual to each cell, of the respective LC member.

5.  Measurement carrier (100) according to claim 4, in which a first transmission line cell (111) has a first predetermined inductance (111a) and further predetermined inductances (112a, 113a) of the further transmission line cells (112, 113) are predetermined as the product of an exponentiation, individual to each cell, of a specified factor and the first predetermined inductance (111a).

6.  Measurement carrier (100) according to any of the preceding claims in which at least one transmission line cell (111, 112, 113) forms a left-hand portion of the measurement transmission line (110).

7.  Measurement carrier (100) according to claim 6, in which the measurement transmission line (110) in its entirety as a series circuit of left-hand and right-hand sections in the form of the respective transmission line cells (111, 112, 113) has a propagation constant of zero in the measurement object-free state for the high-frequency measurement signal.

8.  Measurement carrier (100) according to any of the preceding claims, in which the measurements support surface formed additionally by a second measurement transmission line (210) in electrical parallel connection to the measurement transmission line (110), and which each transmission line cell (111, 112, 113) of the first measurement transmission line (110) is coupled by means of a respective active electronic component, in particular by means of the transistor, with an assigned transmission line cell (211, 212, 213) of the second measurement transmission line (210).

9. Measurement carrier (100) according to any of the preceding claims, in which the measurement transmission line (110) has a protective layer which is designed to protect the material of the measurement transmission line (110) against a mechanical or chemical interaction with the measurement object to be laid thereon.

10. Measuring device (600) for the location-resolved the termination of the dielectric permittivity of a measurement object, comprising

- a control apparatus (660) which is designed to provide a predetermined high-frequency measurement signal;
- an output interface (661) for transmitting the high-frequency measurement signal to a measurement carrier (100) according to any of claims 1 to 9 and an input interface (662) for receiving the high-frequency measurement signal from the measurement carrier (100) which is transmitted by the measurement transmission line (110) of the measurement carrier (100); and
- an evaluating unit (570) which is configured to detect, on the basis of the high-frequency signal received by the input interface (662) when the measurement object is lying in the measuring state, a measurement variable dependent on the propagation constant of the measurement transmission line (110), to compare the detected measurement variable with a sample of the measurement variable determines in the state of the measurement transmission line (110) which it is free of a measurement object, and, on the basis of the comparison results and on the basis of pre-stored parameters, individual to each cell, to specify change components, individual to each cell, of the propagation constant and to determine therefrom permittivity values, individual to each cell, of the measurement object

wherein the pre-stored parameters, individual to each cell, are suitable to specify by calculation a propagation constant, individual to each cell, of the respective transmission line cell (111, 112, 113) in the measurement object-free state, and wherein the propagation constant, individual to each cell, of the respective transmission line cell (111, 112, 113) is different from the respective propagation constants, individual to each cell, of the other transmission line cells (111, 112, 113).

11. Measurement device (600) according to claim 10, in which the control apparatus (660) is configured to provide the high-frequency measurement signal as a sequence of several signals with several different frequencies.

12. Measurement device (600) according to any of claims 10 or 11 which is integrated monolithically with the measurement carrier (100).

13. Method for the location-resolved determination of the permittivity of a measurement object, comprising the steps:

- provision of a measurement carrier (100) according to any of claims 1 to 9;
- laying the measurement object on the support of the measurement carrier (100);
- provision of an electromagnetic high-frequency measurement signal at the measurement transmission line (110) of the measurement carrier (100);
- detection of the high-frequency measurement signal transmitted by the measurement transmission line (110);
- determination of a measurement variable, dependent on the propagation constant of the measurement transmission line (110) on the basis of the detected-frequency measurement signal;
- comparison of the detected measurement variable with a sample of the measurement variable determined in the measurement object-free state of the measurement transmission line (110);
- determination, individual to each cell, of change components in the propagation constant on the basis of the comparison result and on the basis of pre-stored parameters individual to each cell;
- determination of permittivity values, individual to each cell, of the measurement object on the basis of the determined change components individual to each cell.

**Revendications**

1. Support de mesure (100) pour une détermination métrologique à résolution spatiale d'une grandeur de mesure dépendant de la permittivité diélectrique d'un objet de mesure à placer sur le support de mesure (100) comprenant ;

- une base avec une surface d'appui de mesure sur laquelle peut être posé l'objet de mesure,

- une ligne de transmission de mesure (110) qui forme partiellement ou complètement la surface de mesure et qui est configurée en tant que montage électrique en série, constitué d'une pluralité de cellules de ligne de transmission, (111,112, 113) pour transmettre un signal de mesure électromagnétique à haute fréquence pouvant être injecté et ainsi structure la surface de support de mesure de façon cellulaire, dans laquelle

  chacune des cellules de ligne de transmission (111,112, 113) de la ligne de transmission de mesure (110) considérée individuellement

  dans un état exempt de l'objet à mesurer par rapport au signal de mesure haute fréquence, présente une constante de propagation spécifique à la cellule qui diffère des constantes de propagation spécifiques à la cellule respectives des autres cellules de la ligne de transmission (111,112, 113), et est conçue pour adopter une constante de propagation spécifique à la cellule différente dans un état de mesure lorsque l'objet à mesurer repose sur la cellule de ligne de transmission (111,112, 113) que dans l'état sans objet, de sorte que

  une modification de la constante de propagation spécifique à chaque cellule respective de chacune des cellules de la ligne de transmission (111,112, 113) produit une valeur de mesure fonction d'une composante de modification respective et identifiable d'une modification totale sur la base du signal de mesure haut fréquence, capable d'être mesuré, et de la permittivité diélectrique de la ligne de transmission (110) dans son ensemble.

2. Support de mesure (100) selon la revendication 1, dans lequel les cellules de ligne de transmission (111,112, 113) forment chacune un élément LC, et dans lequel la constante de propagation spécifique à la cellule est déterminée à travers une constante de propagation prédéterminée spécifique à la cellule (111b,112b, 113b) de l'élément LC respectif.

3. Support de mesure (100) selon la revendication 2, dans lequel une première cellule de ligne de transmission (111) présente une première capacité prédéterminée (111b) et d'autres capacités prédéterminées (112b, 113b) des autres cellules de ligne de transmission (112, 113) sont déterminées en avance en tant que produit d'une puissance spécifique à la cellule d'un certain facteur et la première capacité prédéterminée (111b).

4. Support de mesure (100) selon l'une des revendications précédentes, dans lequel les cellules de ligne de transmission (111, 112, 113) forment chacune un élément LC, et dans lequel la constante de propagation spécifique à la cellule est obtenue par une inductance prédéterminée (111a,112a, 113a) de l'élément LC respectif.

5. Support de mesure (100) selon la revendication 4, dans lequel une première cellule de ligne de transmission (111) présente une première inductance prédéterminée (111a) et d'autres inductances prédéterminées (112a, 113a) des autres cellules de ligne de transmission (112, 113) sont prédéterminées en tant que produit d'une puissance spécifique à la cellule d'un certain facteur et la première inductance prédéterminée (111a).

6. Support de mesure (100) selon l'une quelconque des revendications précédentes, dans lequel au moins une cellule de ligne de transmission (111, 112, 113) forme une partie gauche de la ligne de transmission de mesure (110).

7. Support de mesure (100) selon la revendication 6, dans lequel la ligne de transmission de mesure (110) dans son ensemble en une connexion en série de tronçons gauche et droit en la forme des cellules de ligne de transmission respectives (111, 112, 113) dans l'état sans objet de mesure pour le signal de mesure haute fréquence présente une constante de propagation de zéro.

8. Support de mesure (100) selon l'une des revendications précédentes, dans lequel la surface de support de mesure est en outre formée par une deuxième ligne de transmission de mesure (210) en parallèle électrique avec la ligne de transmission de mesure (110), et dans lequel chaque cellule de ligne de transmission (111, 112, 113) de la première ligne de transmission de mesure (110) est couplée à une cellule de ligne de transmission associée (211, 212, 213) de la deuxième ligne de transmission de mesure (210) au moyen d'un composant électronique, notamment au moyen d'un transistor.

9. Support de mesure (100) selon l'une des revendications précédentes, dans lequel la ligne de transmission de mesure (110) comporte une couche de protection destinée à protéger le matériau de la ligne de transmission de mesure (110) des interactions mécaniques ou chimiques avec l'objet de mesure à placer.

10. Dispositif de mesure (600) pour la détermination spatialement résolue de la permittivité diélectrique d'un objet de mesure, comprenant

   - un dispositif de commande (660), qui est conçu pour fournir un signal de mesure haute fréquence prédéterminé ;

- une interface de sortie (661) pour transférer le signal de mesure haute fréquence sur un support de mesure (100) selon l'une des revendications 1 à 9 et une interface d'entrée (662) pour recevoir un signal de mesure haute fréquence transmis depuis le support de mesure (100) à travers la ligne de transmission de mesure (110); et

- une unité d'évaluation (570), qui est conçue pour utiliser le signal haute fréquence reçu de l'interface d'entrée (662) lorsque l'objet à mesurer est placé dans l'état de mesure pour détecter une grandeur mesurée qui dépend de la constante de propagation de la ligne de transmission de mesure (110), pour comparer la variable mesurée détectée avec la mesure déterminée lorsque la ligne de transmission de mesure (110) est libre de l'objet de mesure et pour déterminer les composantes de changement spécifiques à la cellule de la constante de propagation sur la base du résultat de la comparaison et basé sur des paramètres spécifiques à la cellule préenregistrés et à partir de ces valeurs pour fournir la permittivité spécifiques à la cellule de l'objet de mesure

dans lequel les paramètres spécifiques à la cellule préenregistrés conviennent pour déterminer mathématiquement une constante de propagation spécifique à la cellule de la cellule de ligne de transmission respective (111, 112, 113) dans l'état sans l'objet à mesurer, et dans lequel la constante de propagation spécifiques à la cellule de la cellule de ligne de transmission respective (111, 112, 113) est différente des constantes de propagation spécifiques à la cellule respectives des autres cellules de ligne de transmission (111, 112, 113).

11. Dispositif de mesure (600) selon la revendication 10, dans lequel le dispositif de commande (660) est agencé pour fournir le signal de mesure haute fréquence sous la forme d'une séquence d'une pluralité de signaux avec une pluralité de fréquences différentes.

12. Dispositif de mesure (600) selon l'une quelconque des revendications 10 ou 11, qui est intégré monolithiquement avec le support de mesure (100).

13. Procédé de détermination spatialement résolue de la permittivité d'un objet de mesure, comprenant les étapes consistant à :

- prévoir un support de mesure (100) selon l'une quelconque des revendications 1 à9 ;
- placer l'objet de mesure sur la base du support de mesure (100) ;

- fournir un signal de mesure électromagnétique haute fréquence à la ligne de transmission de mesure (110) du support de mesure (100) ;
- détecter le signal à fréquence radio transmise par la ligne de transmission de mesure (110) ;
- déterminer le signal de mesure fonction des constantes de propagation de la ligne de transmission de mesure (110) basé sur le signal haute fréquence détecté ;
- comparer la quantité mesurée déterminée avec un échantillon de la quantité mesurée déterminée dans l'état sans objet de mesure de la ligne de transmission de mesure (110) ;
- déterminer spécifiquement à la cellule des composantes de changement de la constante de propagation à l'aide du résultat de la comparaison et à l'aide de paramètres spécifiques à la cellule préenregistrés ;
- déterminer des valeurs de permittivité spécifiques à la cellule de l'objet de mesure sur la base des composantes de changement spécifiques à la cellule déterminés.

**Fig. 1**

**Fig. 2**

**Fig. 3**

**Fig. 4**

S61

S62

S60

S70

# Fig. 5

**Fig. 6**

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20060247896 A
- DE 102006052637 A1